**Europäisches Patentamt**

**European Patent Office**

**Office européen des brevets**

(19)

(11) Numéro de publication : **0 374 591 B1**

(12) **FASCICULE DE BREVET EUROPEEN**

(45) Date de publication du fascicule du brevet :
04.11.92 Bulletin 92/45

(51) Int. Cl.⁵ : **A61K 37/22, A61K 35/78**

(21) Numéro de dépôt : **89122506.2**

(22) Date de dépôt : **06.12.89**

(54) **Inhibition de l'adhésion cellulaire.**

(30) Priorité : **23.12.88 CH 4790/88**

(43) Date de publication de la demande :
**27.06.90 Bulletin 90/26**

(45) Mention de la délivrance du brevet :
**04.11.92 Bulletin 92/45**

(84) Etats contractants désignés :
**AT BE CH DE ES FR GB IT LI LU NL SE**

(56) Documents cités :
**EP-A- 0 092 076**
**EP-A- 0 092 085**

(73) Titulaire : **SOCIETE DES PRODUITS NESTLE S.A.**
**Case postale 353**
**CH-1800 Vevey (CH)**

(72) Inventeur : **Crozier-Willi, Gayle**
**Av. des Vallées 5**
**F-74500 Evian (FR)**
Inventeur : **Fleith, Mathilde**
**30, rue du Lac**
**CH-1800 Vevey (CH)**
Inventeur : **Buchanan, Michael**
**Lake Promenade 417**
**Toronto M8W 1C3 Ontario (CA)**

## Description

L'invention concerne l'utilisation d'un lipide de cassis dans une composition diététique ou pharmaceutique dans le but de prévenir les phénomènes d'adhésion responsables de certaines maladies thrombo-emboliques, inflammatoires et cancéreuses.

On sait que les acides gras polyinsaturés des séries $\omega3$ et $\omega6$ ont des rôles structurels et fonctionnels très importants. Les acides gras polyinsaturés peuvent être désignés par le nombre d'atomes de carbone, le nombre de doubles liaisons et le nombre indiquant la place de la première double liaison comptée à partir du groupe méthyl qui détermine leur famille métabolique, noté $\omega$. Ainsi dans cette nomenclature, l'acide dihomogamma-linolénique (DHLA) est C20:3$\omega$6, l'acide eicosapentaénoïque (EPA) est C20:5$\omega$3 et l'acide arachidonique (AA) est C20:4$\omega$6. Les acides gras linoléique (LA, C18:2$\omega$6) et alphalinolénique (ALA, C18:3$\omega$3) constituent les précurseurs essentiels des autres acides des deux familles qui ne sont pas synthétisés par les mammifères. Aucun métabolisme ne permet de passer d'une famille à l'autre. La conversion de LA ou de ALA en les membres supérieurs respectifs des deux familles est obtenue par désaturation et élongation successives avec des rendements relativement bas.

Les prostaglandines forment une famille de substances qui présentent de nombreux effets biologiques. Le DHLA, l'AA et l'EPA sont transformés sous l'action de la cyclo-oxygénase, en prostaglandines des séries 1(PG1), 2(PG2) et 3(PG3), respectivement. Les PG des séries 1, 2 et 3 comprennent respectivement 1, 2 et 3 doubles liaisons dans leur structure fondamentale d'acides gras à 20 atomes de carbone incluant un groupe cyclopentène. Les PG1 (provenant du DHLA) ont entre autres propriétés celle d'inhiber l'aggrégation des plaquettes sanguines. Par contre les éicosanoïdes de la série 2 (provenant de l'AA), par exemple PG2, thromboxanes, par exemple PxA2 favorisent l'aggrégation plaquettaire. Quant aux eicosanoïdes de la série 3 (provenant de l'EPA), leur rôle est semblable à celui des éicosanoïdes de la série 1.

On suppose que l'efficacité de l'EPA et du DHLA dans la prévention des maladies cardiovasculaires du type thrombo-emboliques est basée sur les effets favorables des PG3 et PG1 par rapport aux effets des PG2.

Par ailleurs, le système enzymatique lipoxygénase conduit aux acides gras hydroxylés et aux leucotriènes à partir des précurseurs AA et EPA. Des études récentes indiquent en particulier que les composés acide 13 -hydroxy-octadécatriènoïque (13-HODE) produit par les cellules endothéliales des vaisseaux sanguins et acide 12 -hydroxy-éicosatétraénoïque (12-HETE) produit par les plaquettes par l'intermédiaire de la lipoxygénase jouent un rôle important dans la médiation de l'adhésion des cellules entre elles et par suite dans la pathogénèse des thromboses, des maladies inflammatoires et de la dissémination des métastases cancéreuses. En simplifiant, l'adhésion serait influencée par l'intermédiaire des régulateurs 12-HETE et 13-HODE, positivement par l'AA, précurseur du 12-HETE et négativement par le LA, conduisant au 13-HODE. Le DHLA est par ailleurs peu susceptible à la lipoxygénase.

On a proposé, par exemple dans la demande de brevet française publiée No 2.553.662, d'ajouter à une composition pharmaceutique ou à un produit alimentaire un mélange d'un premier acide gras choisi parmi l'EPA ou l'acide docosahexaénoïque (DHA) d'une part et d'un deuxième acide gras choisi parmi le DHLA, l'acide cis-linoléique, l'acide gammalinolénique (GLA) d'autre part, dans le but de prévenir les maladies cardiovasculaires. Dans cette demande de brevet, les acides gras en question doivent avoir été isolés séparément à partir de corps gras naturels par iodation suivie d'une saponification, extraction des acides gras par solvant, méthylation de ceux-ci, séparation des esters méthyliques par chromatographie et enfin dé-iodation. L'EPA, par exemple, est obtenu de cette manière à partir de l'huile de foie de morue. La préparation d'une telle composition est particulièrement compliquée.

De plus, l'EPA est particulièrement instable. Enfin, certaines personnes ne supportent pas les réminiscences de mauvais goûts provenant de l'huile de poisson, même désodorisée ou encapsulée.

EP-A-92076 concerne une composition lipidique destinée à l'alimentation orale, entérale ou parentérale contenant une huile fournissant le GLA en combinaison avec un triglycéride à chaîne moyenne et un acide gras à longue chaîne facilitant l'absorption intestinale des lipides. Selon l'antériorité, on devrait s'attendre à une conversion importante des acides gras vers l'AA dans les tissus.

Nous avons trouvé que l'on pouvait par l'utilisation d'un lipide du cassis mettre à disposition l'EPA conjointement au DHLA tout en déprimant la biodisponibilité de l'AA sans avoir les inconvénients associés à l'ingestion d'huile de poisson, par exemple l'augmentation du temps de saignement qui peut provoquer des hémorragies. De cette manière, on obtient des effets très intéressants sur la prévention de l'adhésion et de l'aggrégation des plaquettes, des thromboses et de la dissémination des métastases cancéreuses.

L'invention concerne donc: l'utilisation d'un lipide de cassis pour la fabrication d'une composition diététique ou pharmaceutique pour favoriser la biodisponibilité des acides dihomogammalinolénique et eicosapentaénoïque par rapport à la biodisponibilité de l'acide arachidonique.

Par lipide de cassis, on entend selon l'invention:

-l'huile de pépins de cassis (Ribes nigrum), obtenue par extraction à partir de résidus de cassis et raffinage, par exemple comme indiqué dans le brevet européen No 92.085 ou le brevet européen No 137.862,

-un mélange d'acides gras provenant de l'hydrolyse ou du fractionnement de l'huile de pépins de cassis, obtenus par exemple selon le brevet européen No 178.442 ou selon la demande de brevet européen No 271.747.

-un sel pharmaceutiquement acceptable des acides gras précédents,

-une huile obtenue par réestérification d'un tel mélange d'acides gras avec le glycérol,

-un mélange des lipides précédents.

Le lipide de cassis peut être avantageusement protégé de l'oxydation par un antioxydant liposoluble, par exemple le palmitate d'ascorbyle, les tocophérols, l'acide ascorbique en présence de lécithine ou un mélange de tels antioxydants.

Les compositions diététiques peuvent se présenter sous forme d'émulsions, par exemple des sauces, mayonnaises ou margarines.

Les compositions pharmaceutiques peuvent se présenter sous différentes formes adaptées au mode d'administration, par exemple par voie orale, entérale, rectale ou parentérale. On peut par exemple préparer des capsules, gélules, suppositoires ou sirops. Dans le cas d'une administration entérale ou parentérale, les compositions se présentent sous forme de solutions ou émulsions stabilisées physiquement et chimiquement, apyrogènes et stériles.

La dose administrée dépend du type et de la gravité de l'anomalie à traiter. Elle peut être de 1 à 25 g de lipide de cassis et de préférence de 2 à 5 g d'huile de cassis par jour en prise unique ou de préférence en 2 à 3 prises séparées.

Les exemples ci-après illustrent l'invention. Dans ceux-ci les parties et pourcentages sont pondéraux sauf indication contraire.

Exemple 1

Incorporation de DHLA et de EPA dans les tissus

Beaucoup d'études pharmacologiques sur la faculté de moduler les acides gras des tissus par la diète ont utilisé principalement le rat comme modèle expérimental. On sait que chez le rat ou la souris, l'ingestion de GLA conduit à une augmentation de l'incorporation de GLA dans les triglycérides ou de DHLA dans les lipides totaux, mais pratiquement pas dans les phospholipides. Chez les humains, le lapin et le cochon d'Inde par contre, l'ingestion de GLA conduit à l'incorporation de DHLA dans les triglycérides et les phospholipides du sang, dans la membrane des globules rouges et dans tous les lipides. Pour cette raison le cochon d'Inde a été préféré au rat car, étant beaucoup plus proche de l'homme en ce qui concerne l'activité des désaturases, il reflète mieux la situation qui prévaut chez l'homme. Par ailleurs, le foie a été choisi comme tissu puisqu'il constitue le site majeur des conversions des acides gras.

1.1. Conditions expérimentales

On forme 3 groupes de 7 cochons d'Inde mâles, pesant en moyenne environ 300 g que l'on place dans des cages en macrolon®. On alimente les groupes avec des diètes semi-synthétiques contenant respectivement de l'huile de pépins de cassis (HPC), I, de l'huile de noix (HN), II ou du saindoux (S), III ad libitum pendant 40 jours. L'HN est choisie comme comparaison pour l'HPC car elle ne contient pas les acides GLA et stéaridonique (SA), mais davantage de LA, de sorte que les quantités totales d'acides $\omega6$ et $\omega3$ et leur rapport sont comparables dans les deux huiles.

Tous les animaux ont accès libre à de l'eau contenant 250 mg/l d'acide ascorbique. Après un jeûne d'une nuit, on anesthésie les animaux, on enlève les foies, on les congèle rapidement,on les moud finement et on les entrepose à - 80°C jusqu'à l'analyse. Les compositions de la diète et des lipides ingérés sont indiquées dans les tableaux 1 et 2 ci-après.

TABLEAU 1

Composition de la diète de base
-----------------------------------

Ingrédient %

-----------------------------------

| Caséine | 30 |
| Amidon | 20 |
| Sucrose | 10 |
| Glucose | 3,8 |
| Cellulose | 15 |
| Sels minéraux | 6 |
| Vitamines | 2,2 |
| Acétate de potassium | 2,5 |
| Oxyde de magnésium | 0,5 |
| Lipide | 10 |

TABLEAU 2

Composition des acides gras des lipides des diètes (en moles %)

| Acide gras | HPC | HN | S |
|---|---|---|---|
| C14:0 | - | - | 2,7 |
| C16:0 | 7,4 | 8,6 | 32,4 |
| C16:1 | - | - | 3,4 |
| C17:0 | - | - | 0,5 |
| C17:1 | - | - | 0,3 |
| C18:0 | 0,8 | 1,9 | 14,2 |
| C18:1 $\omega$9 | 10,4 | 20,8 | 36,7 |
| C18:2 $\omega$6 | 48,1 | 57,7 | 8,4 |
| C18:3 $\omega$6 | 17,1 | - | - |
| C18:3 $\omega$3 | 12,7 | 11,0 | 0,7 |
| C18:4 $\omega$3 | 2,6 | - | - |
| C20:1 $\omega$9 | 0,6 | - | 0,7 |
| C20:2 $\omega$6 | 0,3 | - | - |
| | | | |
| Somme des $\omega$6 | 65,5 | 57,7 | 8,4 |
| Somme des $\omega$3 | 15,3 | 11,0 | 0,7 |
| Rapport $\dfrac{\omega 6}{\omega 3}$ | 4,3 | 5,2 | 12,0 |
| Indice d'insaturation[1] | 207,6 | 169,2 | 59,8 |

Légende

1. L'indice d'insaturation est la somme des produits a x b où a est le % molaire de chaque acide gras insaturé et b est le nombre de double liaisons de cet acide gras particulier.

5

## 1.2. Analyse des acides gras

1.2.1.

On prépare les échantillons en homogénéisant 1 g de tissu hépatique congelé dans 20 ml de mélange solvant chloroforme: méthanol de rapport 2:1 en volume et on en extrait les lipides totaux (TL) après séparation des solides déposés par filtration, puis élimination des solvants.

1.2.2.

On sépare les TL en lipides neutres (NL) et en lipides polaires (PL) par passage sur une colonne de gel de silice. A partir des PL, on sépare les fractions non-acide et acide sur colonnes d'échanges d'ions remplies de gel de diéthylaminoéthyl-sépharose. On désale ensuite la fraction de lipides polaires acides par passage sur une colonne de gel de silice.

1.2.3.

On sépare les NL en leurs différents composants par chromatographie haute performance sur couche mince (HPTLC) avec des plaques de verre recouvertes d'acide silicique. On développe les plaques avec un mélange solvant éther de pétrole:diéthyléther:acide acétique de rapport volumique 85:15:0,5, on les sèche à l'air, on les pulvérise avec une solution de 0,005% de primuline dans l'acétone (en poids/volume) et on visualise les bandes correspondant aux différents lipides aux rayons UV. Les esters de cholestérol (CE), triglycérides (TG) et acides gras libres (FFA) ont été identifiés par comparaison avec des produits standards et recueillis dans des tubes de verre fermés avec des bouchons téflonés.

1.2.4.

On sépare les PL en leurs différents composants par HPTLC sur plaques de gel de silice imprégnées avec une solution à 2% (poids/volume) d'acide borique dans le méthanol absolu et on les développe avec un mélange solvant chloroforme:méthanol:triéthylamine:eau de rapport volumique 30:25:34:8. Les phospholipides acides, phosphatidylsérine (PS), phosphatidylinositol (PI), cardiolipine (CL) et les phospholipides non-acides, phosphatidyléthanolamine (PE), et phosphatidylcholine (PC) ont été visualisés comme les lipides neutres, identifiés par rapport aux produits standards et recueillis comme décrit précédemment.

1.2.5.

La composition des TG, CE, FFA et PL est déterminée par chromatographie en phase gazeuse de leurs esters méthyliques. On prépare les esters méthyliques par méthylation de manière connue, puis on les extraits par l'hexane. A partir des chromatogrammes obtenus, on identifie les pics des composés par comparaison avec les chromatogrammes de produits standards.

## 1.3. Résultats

Les résultats d'analyse des lipides sont indiqués dans le tableau 3 ci-après:

TABLEAU 3

Composition des acides gras dans les
classes lipidiques du foie des cochons
d'Inde nourris avec la diète I
(% molaire)

| | | TG | CE | FFA | CL | PI | PS | PC | PE |
|---|---|---|---|---|---|---|---|---|---|
| ω6 | C18:2ω6 | 47,1 | 33,0 | 25,7 | 68,9 | 21,1 | 25,3 | 31,6 | 19,9 |
| | C18:3ω6 (GLA) | 8,1 | 9,1 | 3,6 | 1,5 | 3,2 | 4,0 | 4,5 | 2,7 |
| | C20:3ω6 (DHLA) | 2,3 | 7,0 | 1,4 | 2,1 | 7,5 | 3,6 | 2,7 | 2,3 |
| | C20:4ω6 (AA) | 1,2 | 3,6 | 1,8 | 3,0 | 11,3 | 5,6 | 7,0 | 18,2 |
| ω3 | C18:3ω3 | 9,2 | 5,2 | 4,8 | 5,5 | 1,5 | 1,4 | 2,2 | 1,9 |
| | C18:4ω3 | 0,6 | 0,3 | 0,4 | - | 0,1 | - | 0,1 | - |
| | C20:5ω3 (EPA) | - | 0,2 | - | - | - | - | 0,1 | 0,2 |
| | C22:5ω3 | 0,1 | 0,1 | 0,2 | - | 0,3 | 1,1 | 0,3 | 1,2 |
| | C22:6ω3 | 0,2 | - | 0,7 | 0,2 | 0,3 | 2,2 | 0,7 | 3,5 |

Rapport

$$\frac{C20:3\omega6\ (DHLA)}{C20:4\omega6\ (AA)}$$   1,9   1,9   0,9   0,7   0,7   0,7   0,4   0,1

Indice d'insaturation   178   172   116   190   137   132   136   172

Suite du tableau 3:

Composition des acides gras dans les
classes lipidiques du foie des cochons
d'Inde nourris avec la diète II
(% molaire)

| | | TG | CE | FFA | CL | PI | PS | PC | PE |
|---|---|---|---|---|---|---|---|---|---|
| ω6 | C18:2ω6 | 53,8 | 47,2 | 23,7 | 69,4 | 25,1 | 34,0 | 41,3 | 27,0 |
| | C18:3ω6(GLA) | 0,6 | 0,4 | 0,7 | - | - | 0,1 | - | 0,4 |
| | C20:3ω6(DHLA) | 0,4 | 1,0 | 0,3 | 0,2 | 1,9 | 0,5 | 0,2 | 0,4 |
| | C20:4ω6(AA) | 0,8 | 2,3 | 2,0 | 2,5 | 14,8 | 4,7 | 4,6 | 14,4 |
| ω3 | C18:3ω3 | 9,1 | 6,5 | 4,5 | 4,0 | 1,0 | 1,5 | 2,2 | 1,8 |
| | C18:4ω3 | - | - | 0,1 | - | - | - | - | - |
| | C20:5ω3(EPA) | - | - | - | - | 0,1 | - | - | 0,1 |
| | C22:5ω3 | 0,3 | - | 0,1 | 0,2 | 0,3 | 0,7 | 0,3 | 1,2 |
| | C22:6ω3 | 0,2 | - | 0,9 | 0,6 | 0,4 | 1,8 | 0,9 | 4,5 |

Rapport
$\dfrac{C20:3\omega6(DHLA)}{C20:4\omega6(AA)}$

| TG | CE | FFA | CL | PI | PS | PC | PE |
|---|---|---|---|---|---|---|---|
| 0,5 | 0,5 | 0,2 | 0,1 | 0,1 | 0,1 | $<0,1$ | $<0,1$ |

Indice d'insaturation

| TG | CE | FFA | CL | PI | PS | PC | PE |
|---|---|---|---|---|---|---|---|
| 162 | 150 | 96 | 179 | 133 | 121 | 126 | 167 |

Suite du tableau 3:

Composition des acides gras dans les classes lipidiques du foie des cochons d'Inde nourris avec la diète III (% molaire)

| | TG | CE | FFA | CL | PI | PS | PC | PE |
|---|---|---|---|---|---|---|---|---|
| **ω6** | | | | | | | | |
| C18:2ω6 | 22,4 | 25,5 | 17,7 | 60,4 | 13,7 | 21,1 | 27,5 | 16,0 |
| C18:3ω6(GLA) | 0,2 | 0,1 | 0,6 | – | 0,1 | 0,1 | – | – |
| C20:3ω6(DHLA) | 0,2 | 0,2 | 0,2 | 0,2 | 2,8 | 0,8 | 0,4 | 0,3 |
| C20:4ω6(AA) | 0,7 | 1,7 | 2,7 | 3,5 | 12,7 | 6,7 | 5,2 | 17,5 |
| **ω3** | | | | | | | | |
| C18:3ω3 | 1,5 | 0,8 | 2,1 | 0,5 | 0,1 | 0,5 | 0,6 | 0,3 |
| C18:4ω3 | 0,1 | – | 0,2 | – | – | – | – | – |
| C20:5ω3(EPA) | 0,1 | – | 0,2 | – | 0,4 | 0,2 | 0,3 | 0,7 |
| C22:5ω3 | 0,6 | 0,2 | 0,3 | 0,3 | 0,8 | 1,5 | 0,7 | 2,1 |
| C22:6ω3 | 0,7 | 0,3 | 1,6 | 1,9 | 1,1 | 4,1 | 2,5 | 10,7 |
| Rapport $\frac{C20:3\omega6\ (DHLA)}{C20:4\omega6(AA)}$ | 0,3 | 0,1 | 0,2 | 0,1 | 0,3 | 0,1 | 0,1 | < 0,1 |
| Indice d'insaturation | 104 | 104 | 91 | 170 | 124 | 129 | 119 | 196 |

Les résultats précédents montrent que l'incorporation de GLA et de DHLA est sensiblement plus importante dans le cas du groupe nourri avec la diète I, contenant HPC.

Il n'y a pas de différence notable entre les trois groupes en ce qui concerne l'incorporation de AA. Le résultat de ces deux observations est que le rapport DHLA/AA est sensiblement plus élevé dans le cas de l'ingestion de HPC.

L'incorporation de EPA est sensiblement plus importante dans le cas d'ingestion d'HPC que dans le cas d'ingestion d'HN en ce qui concerne les TG, CE, PC et PE. La même observation est vraie par rapport à l'ingestion de S en ce qui concerne les TG et CE.

L'indice d'insaturation montre que le cochon d'Inde est capable de compenser largerment les différence d'insaturation des acides gras des diètes.

En résumé, les résultats précédents démontrent que l'ingestion d'HPC a pour conséquence une incorporation importante de DHLA et des rapports DHLA/AA plus élevés dans toutes les classes de lipides hépatiques. En outre, l'incorporation d'EPA est plus grande pour le régime à base d'HPC que pour le régime à base d'HN. Il s'en suit que le potentiel des PG1 et PG3 par rapport aux PG2 est clairement démontré en ce qui concerne l'HPC.

Exemple 2

Influence de l'ingestion de HPC sur la composition des acides gras des lipides plasmatiques chez l'homme

On réalise une étude métabolique chez un homme de 32 ans en bonne santé auquel on administre pendant 6 semaines des capsules de concentré d'HPC enrichi en GLA (78%) et en SA (16%) suivant le tableau ci-après:
    jours 1-20: 3 capsules/jour, correspondant à environ 1 g de GLA et 250 mg de SA/jour.
    jours 21-26: 10 capsules/jour, correspondant à environ 3 g de GLA et 0,8 g de SA/jour
    jours 27-37: interruption de l'administration pour cause de grippe
    jours 38-42: 10 capsules/jour, correspondant à environ 3 g de GLA et 0,8 g de SA/jour
L'analyse des acides gras des différentes classes de lipides du sérum sanguin est effectuée en comparant avec le contrôle constitué par la moyenne d'une population recevant un placebo, par chromatographie gazeuse des esters méthyliques.
Les résultats sont indiqués dans le tableau 4 ci-après.

## TABLEAU 4

## Composition des acides gras des différentes classes de lipides sériques en moles % du concentré HPC et du contrôle.

| Acides gras | PL | | FFA | | TG | | CE | |
|---|---|---|---|---|---|---|---|---|
| | Concen-tré HPC | Con-trôle | Concen-tré HPC | Con-trôle | Concen-tré HPC | Con-trôle | Concen-tré HPC | Con-trôle |
| $C20:3\omega6$(DHLA) | 8,35 | 3,83 | 0,93 | 0,48 | 1,60 | 0,38 | 1,97 | 0,88 |
| $C20:4\omega6$(AA) | 16,30 | 14,92 | 1,61 | 1,29 | 3,12 | 1,49 | 11,75 | 9,68 |
| $\dfrac{C20:3\omega6}{C20:4\omega6}$ | 0,51 | 0,26 | 0,58 | 0,02 | 0,51 | 0,25 | 0,17 | 0,09 |
| $C20:5\omega3$(EPA) | 0,94 | 0,68 | - | - | 0,58 | 0,27 | 0,91 | 0,65 |
| $C22:5\omega3$ | 1,52 | 1,28 | 0,87 | 0,19 | 0,90 | 0,50 | 0,18 | - |
| $C22:6\omega3$ | 2,52 | 2,76 | 0,57 | 0,15 | 0,36 | 0,19 | 0,45 | 0,43 |

L'étude des résultats précédents montre que le GLA ingéré est incorporé dans toutes les classes de lipides sériques et est rapidement métabolisé en DHLA et à un degré moindre en AA. On observe une augmentation des acides gras 3 à longue chaîne, en particulier EPA, par rapport au contrôle, ce qui semble indiquer que le SA est également incorporé.
On peut conclure que le potentiel de synthèse des PG1 et PG3 par rapport aux PG2 est grand lorsque le régime est supplémenté en HPC.

Exemple 3

Thrombose et adhésion des plaquettes sanguines

3.1.

Etude de l'incorporation des acides gras dans les parois artérielles. Dans cet exemple, on nourrit des cochons d'Inde avec des diètes contenant différents lipides pendant 6 semaines, puis on détermine la composition des acides gras des aortes des animaux. La composition des acides gras principaux des lipides des diètes est indiquée au tableau 5 et celle des acides gras des aortes au tableau 6 ci-après.

## TABLEAU 5

Composition des acides gras principaux (en moles %) dans les diètes contenant les lipides

|  | HPC | HN | S | HP |
|---|---|---|---|---|
| C14:0 | 0,1 | 0,1 | 1,8 | 3,0 |
| C16:0 | 7,0 | 7,8 | 26,4 | 24,9 |
| C16:1 | 0,1 | 0,1 | 2,1 | 3,3 |
| C18:0 | 1,4 | 2,1 | 15,4 | 12,8 |
| C18:1ω9 | 11,3 | 17,3 | 37,3 | 34,8 |
| C18:2ω6 | 46,0 | 58,5 | 15,4 | 11,9 |
| C18:3ω6 | 16,8 | - | - | 0,1 |
| C18:3ω3 | 14,3 | 13,9 | 1,1 | 2,0 |
| C18:4ω3 | 3,0 | - | 0,2 | 0,4 |
| C20:5ω3 | - | - | 0,1 | 1,7 |
| C22:6ω3 | - | - | 0,2 | 3,2 |
| Somme des ω6 | 63,0 | 58,5 | 12,6 | 12,7 |
| Somme des ω3 | 17,3 | 13,8 | 1,8 | 7,3 |
| Rapport $\frac{\omega 6}{\omega 3}$ | 3,6 | 4,2 | 8,7 | 1,8 |

Légende:

HP = huile contenant 20% d'huile de poisson, 70% de saindoux et 10% d'huile de noix.

S = lipide contenant 90% de saindoux et 10% d'huile de pépins de raisin.

11

TABLEAU 6

Composition des acides gras principaux (en moles %) des aortes des cochons d'Inde nourris avec les diètes contenant les lipides.

|  | HPC | HN | S | HP |
|---|---|---|---|---|
| C18:1$\omega$9 | 20,46 | 27,60 | 43,19 | 41,76 |
| C18:2$\omega$6 | 30,58 | 32,95 | 6,51 | 5,97 |
| C18:3$\omega$6 | 4,47 | 0,33 | 0,12 | 0,03 |
| C18:3$\omega$3 | 6,07 | 4,10 | 0,71 | 0,64 |
| C20:3$\omega$6 (DHLA) | 1,43 | 0,13 | 0,10 | 0,05 |
| C20:4$\omega$6 | 0,97 | 0,62 | 0,91 | 0,91 |
| C20:5$\omega$3 | – | – | – | 0,01 |

On constate une augmentation substantielle du taux de DHLA dans le cas d'ingestion d'HPC. Cela signifie que le potentiel de formation des PG1 est augmenté dans les vaisseaux sanguins.

3.2.

Etude de l'influence de la diète sur les fonctions biologiques des plaquettes sanguines. On mesure la capacité des plaquettes sanguines de cochon d'Inde marquées par la [³H]-adénine d'adhérer à une surface thrombogénique constituée par des disques recouverts de fibronectine après 4 semaines d'ingestion des diètes de l'exemple 3, tableau 5. On compte le nombre de plaquettes ayant adhéré aux disques. Les résultats sont indiqués dans le tableau 7 ci-après.

TABLEAU 7

|  | Diète contenant les lipides | | | |
|---|---|---|---|---|
|  | HPC | HN | S | HP |
| Nombre de plaquettes ayant adhéré sur les disques/cm²x10⁵ | 1,9 | 2,3 | 4,0 | 2,3 |

On constate une diminution de l'adhésion dans le cas de l'ingestion d'HPC par rapport à toutes les autres diètes, y compris celle contenant l'huile de poisson.

3.3.

Etude de la thrombogénicité de la paroi vasculaire et adhésion des plaquettes.

Pour étudier l'influence des acides gras du régime sur la thrombogénicité des vaisseaux sanguins, des groupes de lapins sont nourris avec les diètes semi-synthétiques HPC, HN et HP (tableau 5, exemple 3) et une diète commerciale ("chow", C). Après 4 semaines de régime, on provoque une lésion à l'artère carotide des lapins nourris avec HPC, HN et HP puis on leur injecte des plaquettes sanguines marquées à la [³H]-adénine provenant de lapins donneurs nourris avec la diète C. La mesure de l'accumulation in vivo des plaquettes sur l'artère lésée teste la réactivité du vaisseau sanguin. Pour discriminer l'effet du régime sur la réactivité des plaquettes de l'effet sur la réactivité du vaisseau sanguin, on isole des plaquettes du sang de lapins nourris avec les 4 diètes et on mesure leur adhésion ex vivo sur des disques recouverts de fibronectine.

On détermine également la capacité des vaisseaux sanguins à synthétiser le 13-HODE et celle des plaquettes à synthétiser le 12-HETE.

Les résultats sont indiqués dans les tableaux 8 et 9 ci-après:

## TABLEAU 8

| | | Diète contenant les lipides | | Diète du commerce | |
|---|---|---|---|---|---|
| | | HPC | HN | HP | C |
| Nombre de pla- quettes ayant adhéré sur les disques /cm$^2$x10$^5$ | In vivo | 36 | 52 | 48 | 69 |
| | Ex vivo | 15 | 18 | 9 | 50 |

On constate qu'in vivo la thrombogénicité du vaisseau sanguin est la plus faible avec le régime HPC et qu'il n'y a pas de différence entre les régimes HN et HP. Les mesures ex vivo d'adhésion des plaquettes montrent une réactivité plaquettaire diminuée avec les régimes HPC, HN et HP par rapport au régime C.

TABLEAU 9

| | Diète contenant les lipides | | Diète du commerce | |
|---|---|---|---|---|
| | HPC | HN | HP | C |
| Métabolites de la lipoxygénase | | | | |
| 13-HODE (ng/cm$^2$ de vaisseau) | 30,2 | 27,0 | 4,9 | 12,6 |
| 12-HETE (ng/cm$^2$ plaquettes) | 2,7 | 2,4 | 1,2 | 9,3 |

Légende : ng: manogramme

On n'observe pas de différence dans la synthèse de 12-HETE par les plaquettes des animaux nourris avec les diètes HPC, HN et HP. Par contre la synthèse de 13-HODE par les vaisseaux est plus grande avec les régimes HPC et HN, ce qui concorde avec le contenu plus grand en acide linoléique dans les vaisseaux sanguins de ces deux groupes et avec l'effet antiadhésif du 13-HODE.

Exemple 4

Métastases

4.1.

Pour étudier l'influence de la diète sur la prolifération et la dissémination des métastases, on nourrit des rats pendant 5 semaines avec les diètesde l'exemple 3, tableau 5, à l'exception de celle contenant HN. Un groupe de rats est nourri avec une diète habituelle dite "chow" (C). On cultive des cellules tumorales (Walker 256) dans un milieu contenant le nucléotide radioactif [125 I]-uridine marquant les cellules tumorales. On injecte ensuite les cellules aux rats. On sacrifie les animaux 24 h plus tard et on détermine la radioactivité incorporée dans les poumons.

Les résultats obtenus sont indiqués dans le tableau 10 ci-après.

TABLEAU 10

| | Diète contenant les lipides | | Diète du commerce | |
|---|---|---|---|---|
| | HPC | S | HP | C |
| Nombre de cellules tumorales x 10$^4$ | 3,6 | 13,1 | 1,0 | 8,5 |

On constate que HPC et HP produisent la moindre incorporation de cellules tumorales. On peut penser que cet effet est dû à une interaction entre les cellules tumorales et les cellules endothéliales des parois internes des vaisseaux sanguins: il y a moins d'adhésion des cellules entre elles.

4.2.

On réalise une étude chez le cochon d'Inde à partir de l'essai de l'exemple 3.2, mais appliqué à des animaux auxquels on a préalablement injecté des cellules tumorales. L'injection de cellules tumorales a lieu après 4 semaines d'ingestion des diètes et on continue à donner des diètes aux animaux pendant 3 semaines supplémentaires. On mesure la capacité des vaisseaux sanguins à synthétiser 13-HODE en les incubant avec du LA. On détermine l'adhésion des plaquettes sur les disques comme à l'exemple 3.2. On détermine également le nombre d'animaux ayant des lésions des poumons et le nombre de lésions par poumon. Les résultats sont indiqués dans le tableau 11 ci-après.

## TABLEAU 11

### Diète contenant les lipides

|  | HPC | HN | S | HP |
|---|---|---|---|---|
| Nombre de plaquettes ayant adhéré sur les disques/cm$^2$ x 10$^5$ | 3,6 | 6,6 | 7,1 | 4,1 |
| % de poumons avec des lésions / total | 33 | 40 | 55 | 33 |
| Nombre de lésions par poumon | 8,9 | 9,8 | 21 | 22 |
| % molaire de 13-HODE dans la paroi interne des vaisseaux sanguins | 9,4 | 8,6 | 5,5 | 2,9 |

On constate que comme dans le cas des animaux normaux (exemple 3.2), l'ingestion de HPC comme celle de HP résulte en une diminution de l'adhésion des plaquettes des animaux auxquels on a injecté des cellules tumorales. Le nombre de poumons portant des lésions est le plus faible pour HPC et HP, mais le nombre de lésions par poumon est nettement inférieur pour HPC.

Cependant, une étude histologique a indiqué que les lésions étaient d'origine inflammatoire et non cancéreuse. L'inflammation est donc moindre pour HPC.

Les résultats de l'analyse de 13-HODE montrent une présence accrue de ce médiateur anti-adhésion quand les animaux sont nourris avec l'HPC.

Exemple 5

Inflammation

Les leucocytes polynucléaires (PMN) jouent un rôle déterminant dans le processus inflammatoire et pour cela ils ont besoin de quitter les vaisseaux sanguins pour pénétrer dans les tissus périphériques. Une des étapes conduisant à ce processus est leur adhésion préalable à la paroi interne des vaisseaux sanguins.

On a observé cette adhésion en étudiant l'attraction entre les PNM isolés à partir de cochons d'Inde nourris avec les diètes de l'exemple 3, tableau 5 et des monocouches de cellules endothéliales sur des disques. Les résultats sont indiqués dans le tableau 12 ci-après.

## TABLEAU 12

| | Diète contenant les lipides | | | |
|---|---|---|---|---|
| | HPC | HN | S | HP |
| Nombre de PMN par disque | 1391 | 1938 | 1310 | 2193 |

On voit que l'ingestion de HPC résulte en une faible attraction des PMN aux cellules endothéliales.

## Revendications

1. Utilisation d'un lipide de cassis pour la fabrication d'une composition diététique ou pharmaceutique, pour favoriser la biodisponibilité des acides dihomogammalinolénique et eicosapentaénoïque par rapport à la biodisponibilité de l'acide arachidonique.

2. Utilisation selon la revendication 1, dans une composition diététique ou pharmaceutique destinée à la prévention des maladies d'origine inflammatoire.

3. Utilisation selon la revendication 1, dans une composition diététique ou pharmaceutique destinée à la prévention de l'aggrégation plaquettaire et des thromboses.

4. Utilisation selon la revendication 1, dans une composition diététique ou pharmaceutique destinée à la prévention de la prolifération et de la dissémination des métastases cancéreuses.

## Patentansprüche

1. Verwendung eines Lipides aus schwarzen Johannisbeeren zur Herstellung einer Diätzusammensetzung oder einer pharmazeutischen Zusammensetzung, um die biologische Verfügbarkeit von Dihomogammalinolensäure und von Eicosapentaensäure in bezug auf die biologische Verfügbarkeit der Arachidonsäure zu begünstigen.

2. Verwendung nach Anspruch 1, in einer Diätzusammensetzung oder pharmazeutischen Zusammensetzung, welche zur Vorbeugung gegen Krankheiten entzündlichen Ursprungs bestimmt sind.

3. Verwendung nach Anspruch 1, in einer Diätzusammensetzung oder pharmazeutischen Zusammensetzung, welche zur Vorbeugung gegen eine Plättchenaggregation und Thrombosen bestimmt sind.

4. Verwendung nach Anspruch 1, in einer Diätzusammensetzung oder pharmazeutischen Zusammensetzung, welche zur Vorbeugung gegen eine Wucherung und Ausbreitung von Krebsmetastasen bestimmt sind.

## Claims

1. The use of a blackcurrant lipid for the preparation of a dietetic or pharmaceutical composition for promoting the bioavailability of dihomogammalinolenic acid and eicosapentaenoic acid over the bioavailability of arachidonic acid.

2. The use claimed in claim 1 in a dietetic or pharmaceutical composition intended for the prevention of diseases of inflammatory origin.

3. The use claimed in claim 1 in a dietetic or pharmaceutical composition intended for the prevention of platelet aggregation and thromboses.

4. The use claimed in claim 1 in a dietetic or pharmaceutical composition intended to prevent the proliferation and dissemination of cancerous metastases.